# EUROPEAN PATENT APPLICATION

(11) **EP 4 201 355 A1**
(43) Date of publication of application: **28.06.2023**
(21) Application number: 22215910.5
(22) Date of filing: 22.12.2022
(51) Int. Cl.: A61B 18/14

(54) **COVER FOR FLEXIBLE-CIRCUIT ELECTRODES OF AN ELECTROPHYSIOLOGY BALLOON CATHETER**

(30) Priority: 23.12.2021 US 202163293114 P; 01.12.2022 US 202218073272
(71) Applicant: Biosense Webster (Israel) Ltd, 2066717 Yokneam (IL)
(72) Inventor: BORJIAN, Roozbeh, Irvine 92618 (US); RODRIGUEZ, Jasson, Irvine 92618 (US)
(74) Representative: Carpmaels & Ransford LLP

(57) **Abstract**

Flexible circuit strips of a catheter balloon may comprise a substrate and a contact electrode disposed on the substrate. A cover may be disposed over a peripheral portion of the contact electrode and an adjacent portion of the substrate The cover is intended to increase robustness of the contact electrode in response to fatigue that might arise from repeated expansion and contraction of the catheter balloon.

## Description

### FIELD

The subject matter disclosed herein relates to electrophysiologic catheters, particularly those capable of ablating cardiac tissue via electrodes disposed on a balloon surface.

### BACKGROUND

Ablation of cardiac tissue has been used to treat cardiac arrhythmias. Ablative energies are typically provided to cardiac tissue by a tip portion, which can deliver ablative energy alongside the tissue to be ablated. Some of these catheters administer ablative energy from various electrodes disposed on or incorporated into three-dimensional structures, e.g., wire baskets and balloons.

### SUMMARY OF THE DISCLOSURE

A catheter balloon may be incorporated onto a distal end of a catheter shaft. The catheter balloon may comprise a membrane upon which are disposed flexible circuit strips. The flexible circuit strips may each comprise a substrate including an outer surface and an inner surface, with the inner surface disposed on the membrane. Contact electrodes may be disposed on the outer surfaces of the substrates. A cover may be disposed over a peripheral portion of each contact electrode and an adjacent portion of the corresponding substrate so that the peripheral portion of the contact electrode is not exposed.

The cover is intended to increase robustness of the contact electrode in response to fatigue that might arise from repeated expansion and contraction of the catheter balloon. The cover may comprise an inner edge and an outer edge, while the contact electrode may comprise a boundary disposed between the inner edge and outer edge, such that the cover extends over the boundary. The boundary may be disposed midway between the inner edge and the outer edge. Further, the substrate and the cover may be comprised of a same material, such as polyimide, which may be bonded to the substrate.

The contact electrode may have a fishbone configuration that includes a plurality of protrusions extending away from a main body. As such, the cover may extend from a first protrusion to a second protrusion, or there may be a gap in the cover between the first protrusion and the second protrusion.

### BRIEF DESCRIPTION OF THE DRAWINGS

While the specification concludes with claims, which particularly point out and distinctly claim the subject matter described herein, it is believed the subject matter will be better understood from the following description of certain examples taken in conjunction with the accompanying drawings, in which like reference numerals identify the same elements and in which:
Figure 1 depicts a schematic illustration of an invasive medical procedure;
Figure 2 depicts a top view of a catheter with a balloon in an expanded state, in use with a lasso catheter;
Figure 3 depicts a perspective view of a distal end of the catheter of Figure 2, reflecting the balloon as including a reinforcement comprising a reinforcement component;
Figure 4 depicts a perspective view of a distal end of the catheter of Figure 2, reflecting structure of the catheter internal to the balloon;
Figure 5 depicts a perspective detail view of a strip of a flex circuit electrode assembly on the balloon of Figure 3;
Figure 6 depicts a top view of the strip reflected in Figure 5;
Figure 6A depicts a detailed view of a portion of the strip as depicted in Figure 6;
Figure 6B depicts a cross-section view of a portion of the detailed view depicted in Figure 6A, taken along the section line 6B-6B;
Figure 7A depicts an alternate detailed view of a portion of the strip as depicted in Figure 6; and
Figure 7B depicts a cross-section view of a portion of the detailed view depicted in Figure 7A, taken along the section line 7B-7B.

### MODES OF CARRYING OUT THE INVENTION

The following detailed description should be read with reference to the drawings, in which like elements in different drawings are similarly numbered. The drawings, which are not necessarily to scale, depict selected embodiments and are not intended to limit the scope of the invention. The detailed description illustrates by way of example, not by way of limitation, the principles of the invention. This description will clearly enable one skilled in the art to make and use the invention, and describes several embodiments, adaptations, variations, alternatives and uses of the invention, including what is presently believed to be the best mode of carrying out the invention.

As used herein, the terms "about" or "approximately" for any numerical values or ranges indicate a suitable dimensional tolerance that allows the part or collection of components to function for its intended purpose as described herein. More specifically, "about" or "approximately" may refer to the range of values ±10% of the recited value, e.g. "about 90%" may refer to the range of values from 81% to 99%. In addition, as used herein, the terms "patient," "host," "user," and "subject" refer to any human or animal subject and are not intended to limit the systems or methods to human use, although use of the subject invention in a human patient represents a preferred embodiment.

### Overview

Ablation of cardiac tissue to correct a malfunctioning heart is a well-known procedure. Typically, to successfully ablate, cardiac electropotentials need to be measured at various locations of the myocardium. In addition, temperature measurements during ablation provide data enabling the efficacy of the ablation to be measured. Typically, for an ablation procedure, the electropotentials and the temperatures are measured before, during, and after the actual ablation.

An ablation catheter may include a lumen, and a balloon may be deployed through the catheter lumen. A multi-layer flexible metal structure is attached to an exterior wall or membrane of the balloon. The structure comprises a plurality of electrode groups arranged circumferentially about the longitudinal axis, where each electrode group comprises multiple ablation electrodes, typically arranged longitudinally.

Each electrode group may also include at least one micro-electrode that is insulated physically and electrically from the ablation electrodes in its group. Each electrode group may also include at least a thermocouple. In some embodiments, each electrode group includes a micro-electrode and a thermocouple formed at a common location. Using a single catheter, with the three functionalities of ability to perform ablation, electropotential measurement, and temperature measurement, simplifies cardiac ablation procedures.

### System Description

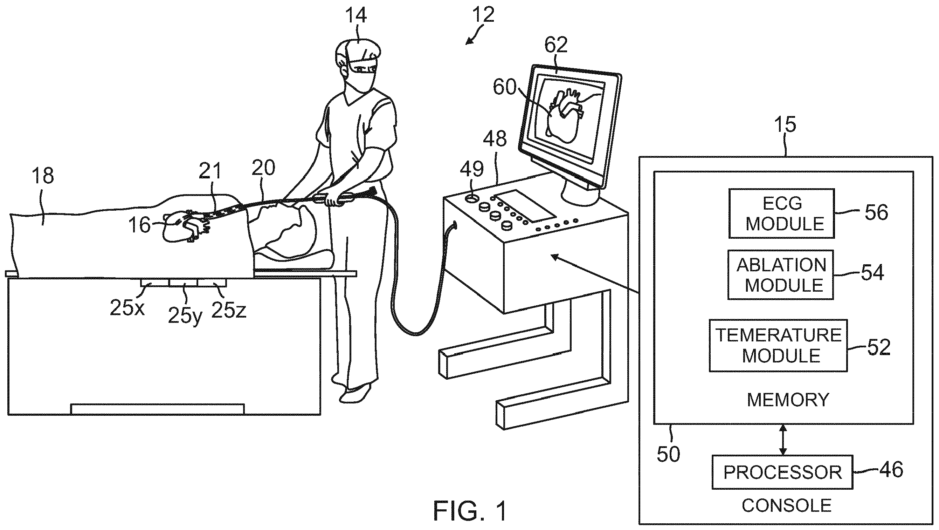

Figure 1 is a schematic illustration of an invasive medical procedure using apparatus 12, according to an embodiment. The procedure is performed by a medical professional 14, and, by way of example, the procedure in the description hereinbelow is assumed to comprise ablation of a portion of a myocardium 16 of the heart of a human patient 18. However, it is understood that embodiments disclosed herein are not merely applicable to this specific procedure, and may include substantially any procedure on biological tissue or on non-biological materials.

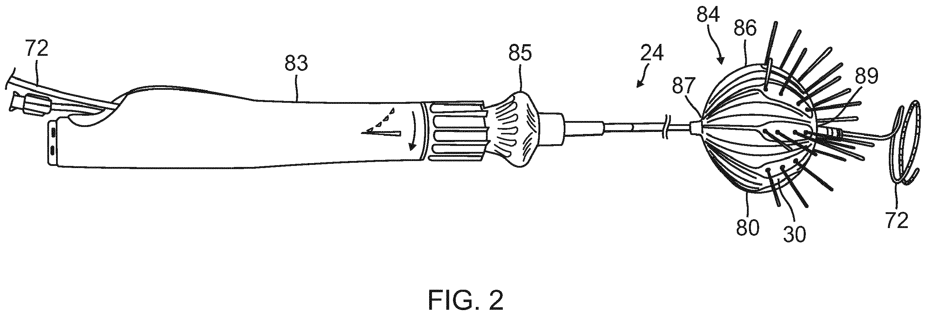

To perform the ablation, medical professional 14 inserts a probe 20 into a sheath 21 that has been pre-positioned in a lumen of the patient. Sheath 21 is positioned so that a distal end 22 of probe 20 enters the heart of the patient. A diagnostic/therapeutic catheter 24 (e.g., a balloon catheter), reflected in Figure 2, is at least partially disposed in a lumen 23 of probe 20, such that it can be deployed through lumen 23 to exit from a distal end of the probe 20.

As shown in Figure 1, apparatus 12 is controlled by a system processor 46, which is in an operating console 15 of the apparatus. Console 15 comprises controls 49 which are used by professional 14 to communicate with the processor. During the procedure, the processor 46 typically tracks a location and an orientation of the distal end 22 of the probe 20, using any method known in the art. For example, processor 46 may use a magnetic tracking method, wherein magnetic transmitters 25X, 25Y and 25Z external to the patient 18 generate signals in coils positioned in the distal end of the probe 20. The CARTOR system (available from Biosense Webster, Inc. of Irvine, California) uses such a tracking method.

The software for the processor 46 may be downloaded to the processor in electronic form, over a network, for example. Alternatively, or additionally, the software may be provided on non-transitory tangible media, such as optical, magnetic, or electronic storage media. The tracking of the distal end 22 is may be displayed on a three-dimensional representation 60 of the heart of the patient 18 on a screen 62. However, it may be displayed two-dimensionally, e.g., by fluoroscopy or MRI

To operate apparatus 12, the processor 46 communicates with a memory 50, which has many modules used by the processor to operate the apparatus. Thus, the memory 50 comprises a temperature module 52, an ablation module 54, and an electrocardiograph (ECG) module 56, the functions of which are described below. The memory 50 typically comprises other modules, such as a force module for measuring the force on the distal end 22, a tracking module for operating the tracking method used by the processor 46, and an irrigation module allowing the processor to control irrigation provided for the distal end 22. For simplicity, such other modules are not illustrated in Figure 1. The modules may comprise hardware as well as software elements. For example, module 54 may include a radio-frequency generator with at least one output or output channel, e.g., ten outputs or ten output channels. Each of the outputs may be separately and selectively activated or deactivated by a switch. That is, each switch may be disposed between the signal generator and a respective output. Thus, a generator with ten outputs would include ten switches. These outputs may each be individually coupled to electrodes on an ablation catheter, e.g., the ten electrodes 33 on balloon 80, described in further detail below. Such an electrical connection may be achieved by establishing an electrical path between each output and each electrode. For example, each output may be connected to a corresponding electrode by one or more wires or suitable electrical connectors. Thus, in some embodiments, an electrical path may include at least one wire. In some embodiments, the electrical path may further include an electrical connector and at least a second wire. Thus, electrodes 33 may be selectively activated and deactivated with the switches to receive radiofrequency energy separately from each of the other electrodes.

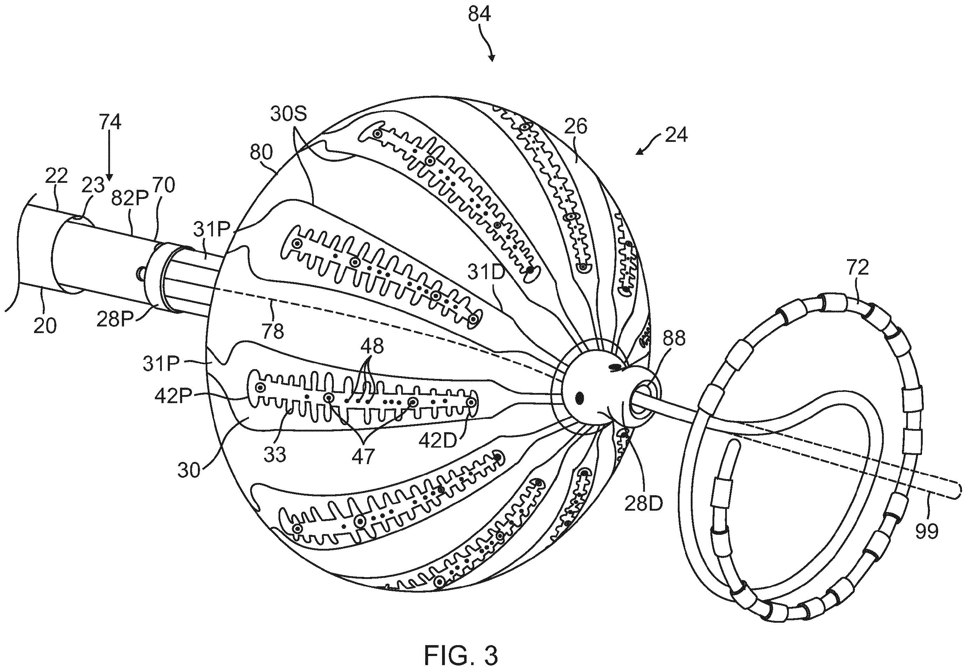

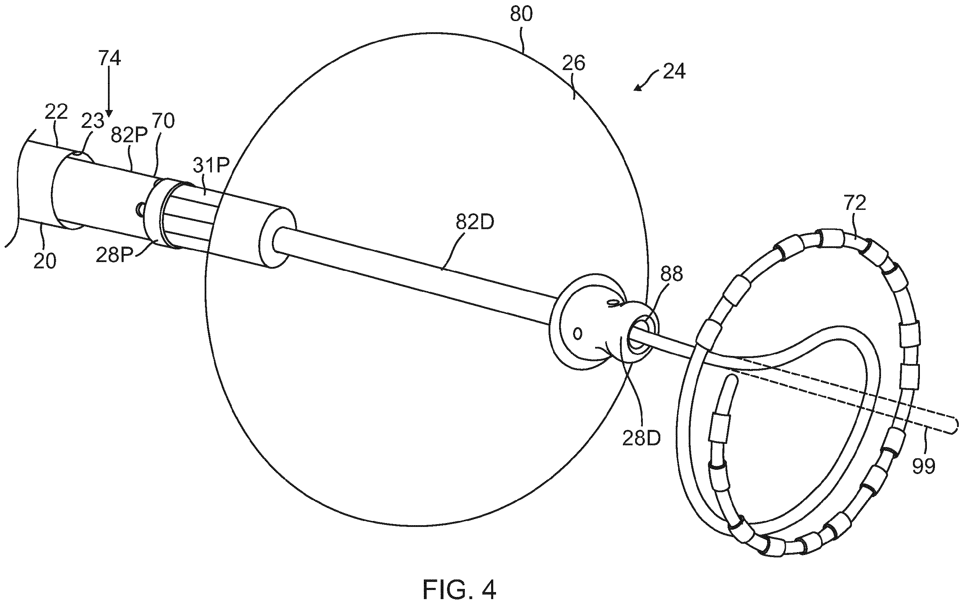

Figure 3 is a schematic perspective view of the diagnostic/therapeutic catheter 24 showing balloon 80 in an expanded configuration. The diagnostic/therapeutic catheter 24 is supported by a tubular shaft 70 having a proximal or first shaft portion 82P, a distal or second shaft portion 82D, and a distal shaft end 88. As depicted in Figure 4, which is similar to Figure 3, but with external structures on balloon 80 hidden, distal or second shaft portion 82D is disposed at least partially within the proximal or first shaft portion 82P in a telescoping relationship therewith. The shaft 70 also includes a hollow central tube 74, which permits a catheter to pass therethrough and past the distal shaft end 88. The catheter may be a focal linear catheter or a lasso catheter 72, as illustrated. The lasso catheter 72 may be inserted into the pulmonary vein to position the diagnostic/therapeutic catheter 24 correctly with respect to an ostium prior to ablation of the ostium. The distal lasso portion of the catheter 72 is typically formed of shape-memory retentive material such as nitinol. It is understood that the diagnostic/therapeutic catheter 24 may also be used with a linear or focal catheter 99 (as shown in broken lines in Figure 3) in the PV or elsewhere in the heart. The focal catheter 99 may include a force sensor at its distal tip. Suitable force sensing distal tips are disclosed in U.S. Patent Nos. 8,357,152 and 10,688,278, the entire contents of which are incorporated by reference herein. Any catheter used in conjunction with the diagnostic/therapeutic catheter may have features and functions, including, for example, pressure sensing, ablation, diagnostic, e.g., navigation and pacing.

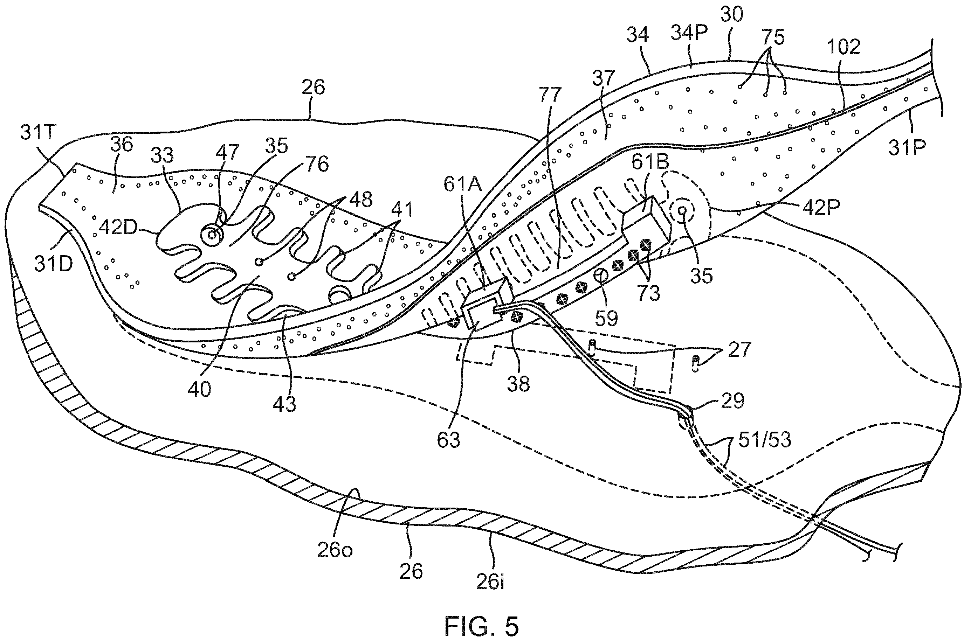

With further reference to Figure 5, balloon 80 of the diagnostic/therapeutic catheter 24 comprises a membrane 26 of a bio-compatible material, for example, formed from a plastic such as polyethylene terephthalate (PET), polyurethane, Pellethane^{®} or PEBAX^{®}. Membrane 26 has an outer surface 26o and an inner surface 26i. Membrane 26, and thus balloon 80, have a proximal end 87 and a distal end 89 at distal shaft end 88.

The shaft 70 and the distal shaft end 88 define a longitudinal axis 78 of the balloon 80. The balloon 80 is deployed, in a collapsed configuration, via the lumen 23 of the probe 20. A proximal end of membrane 26 of balloon 80 is attached to first or proximal shaft portion 82P and a distal end of membrane 26 of balloon 80 is attached to second or distal shaft portion 82D, proximate to distal shaft end 88. Balloon 80 may be expanded to an expanded configuration after exiting from the distal end 22 by moving distal shaft end 88 proximally to shorten the distance between the distal end 89 of balloon 80 and proximal end 87 of balloon 80, and thus increase the width of balloon 80, i.e., by telescoping distal shaft portion 82D proximally in the proximal shaft portion 82P. Passing irrigation fluid into balloon 80 may further expand balloon 80. Balloon 80 may be returned to its collapsed configuration by ceasing the irrigation and then moving distal shaft end 88 away from proximal end 87 to decrease the width of and extend the length of balloon 80, i.e., by telescoping distal shaft portion 82D distally in proximal shaft portion 82P. This telescopic motion between the first shaft portion and the second shaft portion may be controlled by knob 85 of control handle 83, shown in Figure 2. Specifically, knob 85 may be rotated in a first direction to extend distal shaft portion 82D distally, and thus move distal shaft end 88 distally, whereas knob 85 may be rotated in the opposite direction to withdraw distal shaft portion 82D proximally, and thus move distal shaft end 88 proximally. Knob 85 may further include locking features, such as detents, to maintain distal shaft end at its most proximal location and at its most distal location. Such locking features help prevent balloon 80 from collapsing somewhat out of its expanded configuration during ablation and from expanding somewhat out if its collapsed configuration during withdrawal into lumen 23 of probe 20. Further description concerning transitioning the balloon between a collapsed configuration and an expanded configuration is set forth in U.S. Patent Application No. 15/827,111, published as U.S. Patent Application Publication No. 2018/0161093. The entire content of this application is incorporated by reference herein in its entirety.

The membrane 26 supports and carries a combined electrode and temperature sensing member which is constructed as a multi-layer flexible circuit electrode assembly 84. The "flex circuit electrode assembly" 84 may have many different geometric configurations. In the illustrated embodiment, the flex circuit electrode assembly 84 has a plurality of radiating flexible-circuit strips 30. The strips 30 are evenly distributed about outer membrane surface 26o of balloon 80. Each comprises a substrate 34 that has wider proximal portion that gradually tapers to a narrower distal portion. As seen in Figure 3, flexible circuit electrode assembly 84 may include ten strips 30.

Each substrate 34 has a proximal tail 31P proximal to the wider proximal portion and a distal tail 31D distal of the narrower distal portion. As described below, distal tail portion 31D may extend up to distal shaft end 88 to be secured thereunder. Substrate 34 may be bonded to membrane 26 with an adhesive, such as an epoxy. Some adhesive may be disposed between inner surface 37 of substrate 34 and membrane 26.

The flex circuit electrode assembly 84 is described with respect to one of its strips 30 as shown in Figure 5. The flex circuit electrode assembly 84 includes a flexible and resilient sheet substrate 34, constructed of a non-electrically conductive bio-compatible material, for example, polyimide. In some embodiments, the sheet substrate 34 has a greater heat resistance (or a higher melting temperature) compared to that of the balloon membrane 26. In some embodiments, the substrate 34 is constructed of a thermoset material having a decomposition temperature that is higher than the melting temperature of the balloon membrane 26 by approximately 100 degrees Celsius or more. The substrate may have a thickness of between about 15 micrometers to about 25 micrometers, e.g., about 20 micrometers.

The substrate 34 is formed with one or more irrigation pores or apertures 35 that are in alignment with the irrigation apertures 27 of the balloon member 26 so that fluid passing through the irrigation apertures 27 and 35 can pass to the ablation site on the ostium. Substrate 34 may be cut to shape by, and the irrigation pores 35 formed by, any suitable manufacturing technique, such as laser cutting.

The substrate 34 has a first or outer surface 36 facing away from the balloon membrane 26, and a second or inner surface 37 facing the balloon membrane 26. On its outer surface 36, the substrate 34 supports and carries the contact electrodes 33 that may be exposable to an environment, such as an interior of a portion of a heart, and particularly adapted for tissue contact with a pulmonary vein ostium. On its inner surface 37, the substrate 34 supports and carries a wiring electrode 38. The contact electrode 33 delivers RF energy to the ostium during ablation or is connected to a thermocouple junction for temperature sensing of the ostium. In the illustrated embodiment, the contact electrode 33 has a longitudinally elongated portion 40 and a plurality of thin transversal linear portions or protrusions 41 extending generally perpendicularly from each lateral side of the elongated portion 40 between enlarged proximal and distal ends 42P and 42D, generally evenly spaced therebetween. The elongated portion 40 has a greater width and each of the protrusions has a generally uniform lesser width. Accordingly, the configuration or trace of the contact electrode 33 may resemble a "fishbone" with protrusions 41 being "fish bones" of varying lengths extending from a main body of the electrode, but it should be noted that the invention is not limited to such configuration. In contrast to an area or "patch" ablation electrode, the protrusions 41 of the contact electrode 33 advantageously increase the circumferential or equatorial contact surface of the contact electrode 33 with the ostium while void regions 43 between adjacent protrusions 41 advantageously allow the balloon 80 to collapse inwardly or expand radially as needed at locations along its equator. In the illustrated embodiment, the protrusions 41 have different lengths, some being longer, others being shorter. For example, the plurality of protrusions includes a distal protrusion, a proximal protrusion and protrusions therebetween, where each of the protrusions in between has a shorter adjacent protrusion. For example, each protrusion has a length different from its distal or proximal immediately adjacent neighboring protrusion(s) such that the length of each protrusion generally follows the tapered configuration of each substrate 34. In the illustrated embodiment, there are 22 protrusions extending across (past each lateral side of) the elongated portion 40, with the longest protrusion being the third protrusion from the enlarged proximal end 42P. In some embodiments, contact electrode 33 includes an outer or contact layer 76 and an inner or seed layer 77 between contact layer and substrate 34. Contact layer 76 may comprise gold. Seed layer 77 may comprise titanium, tungsten, palladium, silver, or combinations thereof. Seed layer 77 may be sputtered onto substrate 34 and contact layer 76 may be sputtered, plated, or a combination thereof, onto the seed layer. The thickness of seed layer 77 may be between about 250 angstroms to about 350 angstroms, e.g., about 300 angstroms. The thickness of contact layer 76 may be between about .75 micrometers to about 1.25 micrometers, e.g., about 1 micrometer. Whatever material seed layer 77 comprises, e.g., titanium, it should be readily bondable to the material of substrate 34, e.g., polyimide.

Formed within the contact electrode 33 are one or more exclusion zones 47, each surrounding an irrigation aperture 35 formed in the substrate 34. The exclusion zones 47 are voids purposefully formed in the contact electrode 33 so as to avoid damage to the contact electrode 33 during construction of the electrode assembly 84 in accommodating the irrigation apertures 35 at their locations and in their function.

Also formed in the contact electrode 33 are one or more conductive blind vias 48 which are conductive or metallic formations that extend through through-holes in the substrate 34 and are configured as electrical conduits connecting the contact electrode 33 on the outer surface 36 and the wiring electrode 38 on the inner surface 37. It is understood that "conductive" is used herein interchangeably with "metallic" in all relevant instances. For example, the via may be fabricated by plating gold through the through-holes in substrate 34. Preferably, the thickness blind via 48 is less than or approximates the thickness of substrate 34. For example, the thickness of blind via 48 may be between about 4 micrometers and about 25 micrometers, e.g., about 10 micrometers to about 14 micrometers.

In the illustrated embodiment, the contact electrode 33 measures longitudinally between about 0.1 inch and 1.0 inch, and preferably between about 0.5 inch and 0.7 inch, and more preferably about 0.57 inch, and has four exclusion zones 47 and nine blind vias 48.

On the inner surface 37 of the substrate 34, the wiring electrode 38 is generally configured as an elongated body generally similar in shape and size to the elongated portion 40 of the contact electrode 33. The wiring electrode 38 loosely resembles a "spine" and also functions as a spine in terms of providing a predetermined degree of longitudinal rigidity to each substrate 34 of the electrode assembly 84. The wiring electrode 38 is positioned such that each of the blind vias 48 is in conductive contact with both the contact electrode 33 and the wiring electrode 38. In the illustrated embodiment, the two electrodes 33 and 38 are in longitudinal alignment with other, with all nine blind vias 48 in conductive contact with both electrodes 33 and 38. In some embodiments, the wiring electrode 38 has an inner portion of copper and a peripheral portion of gold.

The wiring electrode 38 is also formed with its exclusion zones 59 around the irrigation apertures 35 in the substrate 34. The wiring electrode 38 is further formed with solder pad portions, at least one active 61A, and there may be one or more inactive solder pad portions 61B. The solder pad portions 61A and 61B are extensions from a lateral side of the elongated body of the wiring electrode 38. In the illustrated embodiment, an active solder pad portion 61A is formed at about a mid-location along the elongated body, and a respective inactive solder pad portion 61B is provided at each of the enlarged distal end 42D and the enlarged proximal end 42P.

Attached, e.g., by a solder weld 63, to the active solder pad portion 61A are the wire pair, e.g., a constantan wire 51 and a copper wire 53. The copper wire 53 provides a lead wire to the wiring electrode 33, and the copper wire 53 and the constantan wire 51 provide a thermocouple whose junction is at solder weld 63. The wire pair 51/53 are passed through a through-hole 29 formed in the membrane 26. It is understood that, in other embodiments in the absence of the through-hole 29, the wire pair 51/53 may run between the membrane 26 and the substrate 34 and further proximally between the membrane 26 and the proximal tail 31P until the wire pair 51/53 enters the tubular shaft 70 via another through-hole (not shown) formed in the tubular shaft sidewall closer to the proximal ring 28P.

The substrates 34 are affixed to the balloon membrane 26 such that the outer surface 36 of the substrate 34 is exposed and the inner surface 37 of the substrate 34 is affixed to the balloon membrane 26, with the wiring electrode 38 and wire pair 51/53 sandwiched between the substrate 34 and the balloon membrane 26. The irrigation apertures 35 in the substrate 34 are aligned with the irrigation apertures 27 in the balloon membrane 26. The exclusion zones 59 in the wiring electrode 38 and the exclusion zones 47 in the contact electrode 33 are concentrically aligned with each other, as well as with the irrigation apertures 27 and 35 in balloon 26 and substrate 34, respectively.

Further details on constructing a diagnostic/therapeutic catheter in accordance with the foregoing disclosure may be found in U.S. Patent Application No. 15/360,966, published as U.S. Patent Application Publication No. 2017/0312022. The entire content of this application is incorporated by reference herein in its entirety.

### Improved Robustness

Through ongoing research and product development efforts concerning the subject matter described above, Applicant has determined that balloon 80 must be able to withstand multiple cycles of being deployed from lumen 23 of probe 20 in a collapsed configuration, expanded to an expanded configuration, returned to the collapsed configuration, and withdrawn into lumen 23 of probe 20. The number of cycles may be from about five to about twenty. Thus, the overall integrity of the assembled balloon must withstand at least five to twenty fatigue cycles.

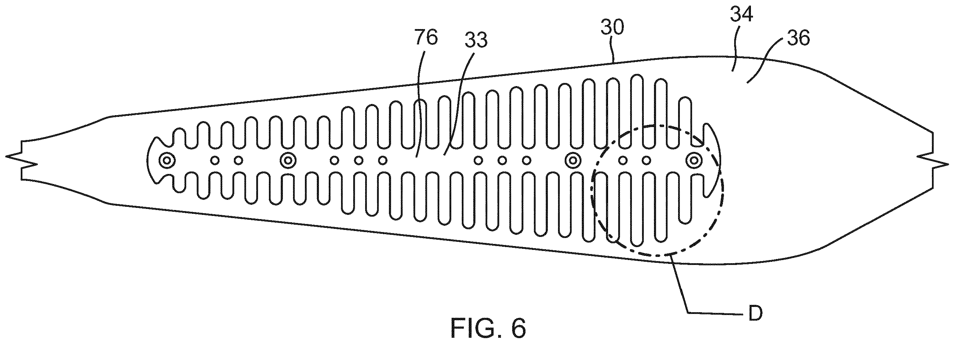

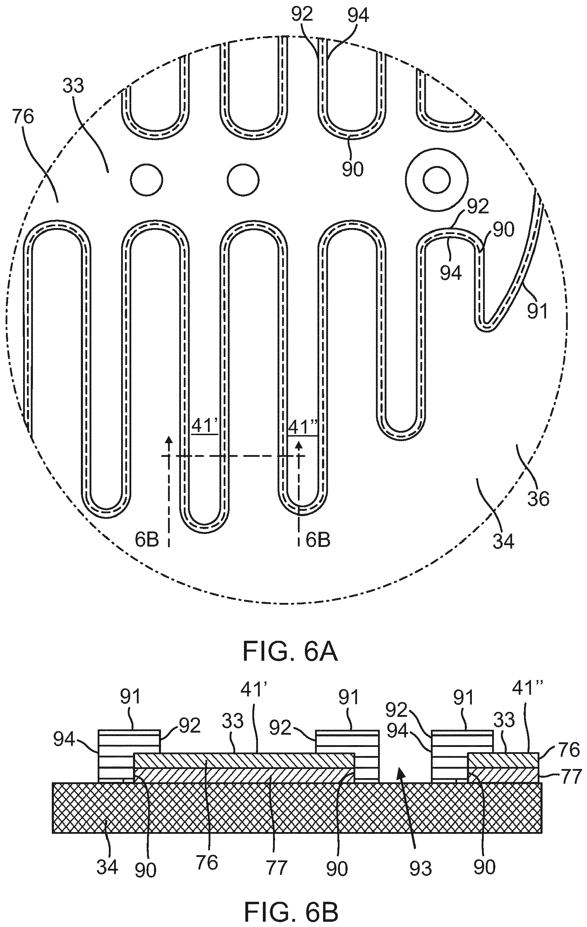

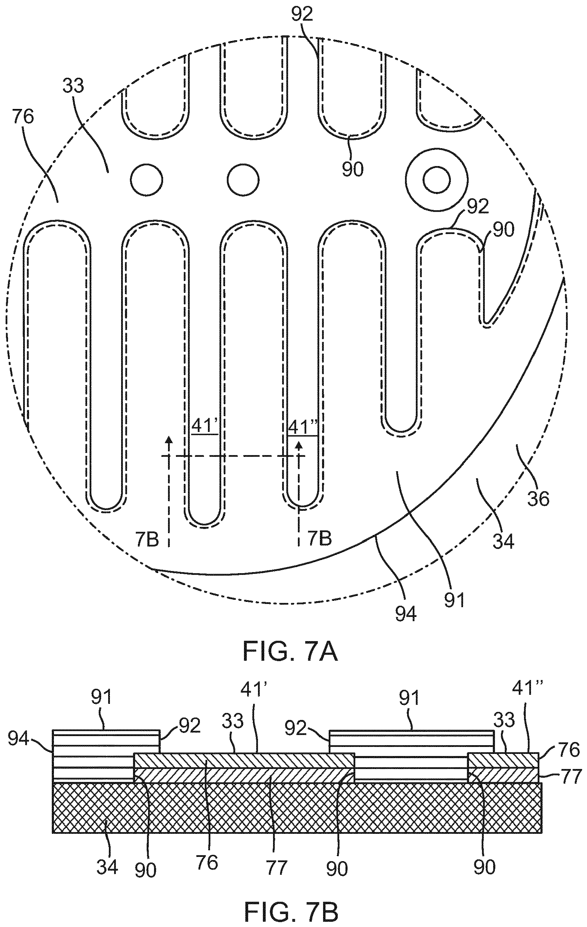

During these efforts, Applicant has identified an opportunity for ensuring the robustness of contact electrode 33 without comprising its function or compromising the integrity of balloon 80 during its multiple cycles of deployment. Specifically, as balloon 80 is repeatedly deployed and retracted, a very rare possibility arises that contact layer 76 may begin to be scraped or delaminated from seed layer 77. When such delamination occurs, contact layer 76 may crack or flake away from seed layer 77 at boundary 90 of contact electrode 33, primarily along protrusions 41. Although Applicant has determined that such delamination may be avoided by ensuring that boundary 90 has smooth edges, Applicant has also determined another technique for avoiding such delamination. This technique is described with reference to Figure 6 and its detail D, corresponding to the alternate detail views depicted in Figures 6A and 7A, and their respective cross-sections depicted in Figures 6B and 7B.

Figure 6 depicts a top view of a single strip 30 of flex circuit 84, including contact electrode 33 and substrate 34. Again, contact electrode 33 includes a contact layer 76, which may be gold and have a fishbone configuration, whereas substrate 34 may be polyimide. Boundary 90 of contact electrode 33 is indicated by the dotted line in the detail view of Figure 6A and Figure 7A. Whenever the aforementioned delamination has been observed by Applicant, it has typically begun at boundary 90. Based on this observation, Applicant has determined that such delamination may be prevented by covering a peripheral portion of contact electrode 33 with a cover 91 that extends over boundary 90 and onto an adjacent portion of substrate 34. Specifically, as depicted in Figure 6A and Figure 7A, cover 91 may be disposed over boundary 90, such that cover 91 has an inner edge 92 atop contact layer 76 of contact electrode 33 and an outer edge 94 atop outer surface 36 of substrate 34. Referring to the cross section of Figure 6B as indicated by the section line 6B-6B in Fig. 6A, it can be seen that contact electrode 33, comprising contact layer 76 and seed layer 77, is partially sandwiched by cover 91 with inner edges 92 disposed atop contact layer 76. In this configuration, cover 91 can include a gap 93 between adjacent protrusions, such as first protrusion 41' and second protrusion 41". Accordingly, a peripheral portion of contact electrode 33, i.e., the portion between cover inner edge 92 and contact electrode boundary 90, becomes sandwiched between cover 91 and substrate 34, while the portion of cover 91 between outer edge 94 and boundary 90 covers the adjacent portion of substrate 34.

Inner edge 92 of cover 91 should be positioned atop contact electrode 33 at a sufficient distance away from boundary 90 to ensure that cover 91 minimizes any likelihood of delamination without interfering with the ability of the contact electrode to properly ablate tissue. Thus, for example, a covering width, i.e., a width between boundary 90 and inner edge 92, may be between about 0.005 inchers and about .02 inches, e.g., about 0.013 inches.

While the location of outer edge 92 on substrate 34 need not be defined based on ablation functionality, providing more of cover 91 atop substrate 34 than is necessary to ensure a robust attachment of cover 91 to substrate 34 may inhibit other important characteristics of balloon 80, such as its conformance characteristics. For example, the overall width of cover 91 may be between about 0.01 inches to about .04 inches, e.g., about .025 inches. However, the width of cover 91 need not necessarily be precisely defined, nor include gap 93, as indicated in the preceding paragraph. Instead, referring to the detail view of Figure 7A and the cross-section view of Figure 7B, cover 91 may be configured to extend continuously over the space between adjacent protrusions 41, such as the space between protrusion 41' and 41", as well as to extend further out onto substrate 34 than indicated in Figure 6A, e.g., covering the entirety of the portion of substrate 34 that is not covered by contact electrode 33. As such, there is no gap 93 between adjacent protrusions. Correspondingly, outer edge 94 is nowhere disposed between neighboring protrusions, e.g., first protrusion 41' and second protrusion 41". Thus, the embodiment depicted in Figures 7A and 7B allows for a structurally more robust cover 91 to extend from one electrode contact surface to another electrode contact surface without interruption.

Cover 91 may comprise the same material as the substrate, e.g., polyimide. Polyimide is a preferred material for preventing delamination of a gold contact layer 76 because polyimide bonds readily to itself and to gold. Cover 91 may be applied by any suitable process, such as sputtering, lamination, or vapor deposition. A first mask may be provided over the entirety of contact layer 76 except for the portion of contact layer 76 between inner edge 92 and boundary 90. A second mask may be provided over portions of substrate 34 to define a location of outer edge 94. Thus, upon application of cover 91, boundary 90 is disposed under cover 91, between inner edge 92 and outer edge 94, e.g. midway between inner edge 92 and outer edge 94 as reflected in Figure 6A. The thickness of cover 91 may be between about 3 micrometers and about 7 micrometers, e.g., about 5 micrometers.

Any of the examples or embodiments described herein may include various other features in addition to or in lieu of those described above. The teachings, expressions, embodiments, examples, etc., described herein should not be viewed in isolation relative to each other. Various suitable ways in which the teachings herein may be combined should be clear to those skilled in the art in view of the teachings herein.

Having shown and described exemplary embodiments of the subject matter contained herein, further adaptations of the methods and systems described herein may be accomplished by appropriate modifications without departing from the scope of the claims. In addition, where methods and steps described above indicate certain events occurring in certain order, it is intended that certain steps do not have to be performed in the order described but in any order as long as the steps allow the embodiments to function for their intended purposes. Therefore, to the extent there are variations of the invention, which are within the spirit of the disclosure or equivalent to the inventions found in the claims, it is the intent that this patent will cover those variations as well. Some such modifications should be apparent to those skilled in the art. For instance, the examples, embodiments, geometrics, materials, dimensions, ratios, steps, and the like discussed above are illustrative. Accordingly, the claims should not be limited to the specific details of structure and operation set forth in the written description and drawings.

## Claims

1. A catheter balloon, comprising:
a membrane including a proximal end and a distal end;
a plurality of flexible circuit strips disposed about the membrane, the flexible circuit strips each comprising:
a substrate including an outer surface and an inner surface, the inner surface disposed on the membrane,
a contact electrode disposed on the outer surface of the substrate so that the contact electrode is exposable to an environment; and
a cover disposed over a peripheral portion of the contact electrode and an adjacent portion of the substrate so that the peripheral portion of the contact electrode is no longer exposed.

2. The catheter balloon of claim 1, in which the cover comprises and inner edge and an outer edge, and the contact electrode comprises a boundary disposed between the inner edge and outer edge, such that the cover extends over the boundary.

3. The catheter balloon of claim 2, in which the boundary is disposed midway between the inner edge and the outer edge.

4. The catheter balloon of claim 3, in which the substrate and the cover are comprised of a same material.

5. The catheter balloon of claim 4, in which the same material comprises polyimide.

6. The catheter balloon of claim 2, in which the cover is bonded to the substrate.

7. The catheter balloon of claim 2, in which a covering width between the inner edge of the cover and the boundary of the contact electrode is between about 0.005 inches and about 0.02 inches.

8. The catheter balloon of claim 7, in which the covering width is about 0.013 inches.

9. The catheter balloon of claim 7, in which an overall width of the cover is between about 0.01 inches to about 0.04 inches.

10. The catheter balloon of claim 9, in which the overall width is about 0.025 inches.

11. The catheter balloon of claim 7, in which a thickness of the cover is between about three micrometers and about seven micrometers.

12. The catheter balloon of claim 11, in which the thickness is about five micrometers.

13. The catheter balloon of claim 2, in which the contact electrode comprises a contact layer and a seed layer.

14. The catheter balloon of claim 13, in which the seed layer is disposed on the outer surface of the substrate.

15. The catheter balloon of claim 14, in which the seed layer comprises titanium.

16. The catheter balloon of claim 14, in which a thickness of the seed layer is between about 250 angstroms and about 350 angstroms.

17. The catheter balloon of claim 16, in which the thickness of the seed layer is about 300 micrometers.

18. The catheter balloon of claim 13, in which the cover is disposed over a peripheral portion of the contact layer.

19. The catheter balloon of claim 18, in which a thickness of the contact layer is between about 0.75 micrometers and about 1.25 micrometers.

20. The catheter balloon of claim 19, in which the thickness of the contact layer is about one micrometer.
